(19) Europäisches Patentamt
European Patent Office
Office européen des brevets

(11) EP 3 928 761 A1

# (12) EUROPEAN PATENT APPLICATION

(43) Date of publication:
**29.12.2021 Bulletin 2021/52**

(21) Application number: **20382555.9**

(22) Date of filing: **24.06.2020**

(51) Int Cl.:
***A61K 8/58*** *(2006.01)* ***A61K 8/898*** *(2006.01)*
***A61Q 5/00*** *(2006.01)* ***A61Q 5/04*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Revlon Consumer Products Corporation**
**New York, NY 10004 (US)**

(72) Inventors:
- **BEATO MERINO, Raquel**
  **08027 Barcelona (ES)**
- **FERNÁNDEZ REYES, Sandra**
  **08014 Barcelona (ES)**
- **VALLECILLOS LÓPEZ, Rocío**
  **08207 Barcelona (ES)**

(74) Representative: **Gallardo, Antonio M.**
**Revlon**
**Tirso de Molina, 40, Ed. 4**
**08940 Cornellà de Llobregat (ES)**

## (54) COMPOSITIONS FOR TREATING KERATIN FIBERS

(57) Compositions for treating keratin fibers are described. Said compositions comprise, in a cosmetically acceptable medium: (a) at least one film-forming amino-silicone polymer, and (b) at least one silane derivative of a hydrolyzed protein or peptide or amino acid. These compositions provide an anti-frizz effect to hair while reinforcing and repairing hair as well as protecting hair during heat treatments (heat-protection).

A process for treating keratin fibers using these compositions and the use of these compositions for protecting keratin fibers from heat damage are also described.

EP 3 928 761 A1

## Description

### TECHNICAL FIELD

Compositions for treating keratin fibers are described. These compositions provide an anti-frizz effect to hair while reinforcing and repairing hair as well as protecting hair from damage during heat treatments (heat-protection).

### BACKGROUND

The information provided below is not admitted to be prior art to the present disclosure, but is provided solely to assist the understanding of the reader.

Human hair is structured in highly organized strata that are very resistant to external stimuli. Its high stability is due to stiffness of the central $\alpha$-helical core of keratin and to a high number of disulfide crosslinks.

Although weather and oxidants (i.e., environmental factors) can give rise to hair damage, morphological changes can result from daily care routines. In particular, permanent wavings, straightening or relaxing and bleaching during hair coloring processes are major causes of hair damage. These alterations results in poor manageability, dryness, brittleness, loss of shine and decreased strength (fiber breakage).

Heat-assisted styling of human hair, particularly using blow dryers, hair straighteners (flat irons), curling devices, heated combs, heated brushes (with or without a rotating drum), or other heating means, can also damage hair by subjecting the hair to too much heat.
The overheating causes moisture to evaporate or be driven out of the hair so that the hair becomes brittle and more susceptible to cracking. In addition, overheating in heat styling can cause physical damage to the hair, particularly by raising the cuticles and/or creating blisters on individual hair fibers, thus causing increased friction between the hair fibers. As a result, hair develops a positive electrostatic charge. The positive charge of individual hair fibers causes the hair fibers to repel one another resulting in a "static flyaway" condition (i.e., frizzy hair), making it more difficult to comb, requiring increased force to comb the hair, which in turn can wear the outer surface of the hair and cause cracks and breaks in the hair.

There is therefore a need for hair treatments that can provide an improvement of the fatigue lifetime of hair, especially hair that has been subjected to adverse conditions or treatments, particularly hair treatments that provide an anti-frizz effect to hair as well as protecting hair from damage during heat treatments (heat-protection), and to do so in a long-lasting manner.

### SUMMARY

Described herein is a composition for treating keratin fibers comprising, in a cosmetically acceptable medium, (a) at least one film-forming amino-silicone polymer, and (b) at least one silane derivative of a hydrolyzed protein or peptide or amino acid.

This composition can provide an anti-frizz effect to hair as well as protect hair from damage during heat treatments (heat-protection), and do so in a long-lasting manner, i.e. at least for 24 hours.

In an embodiment, in a composition as defined above, the at least one film-forming amino-silicone polymer is selected from Polysilicone-35, Polysilicone-33, Polysilicone-29, Polysilicone-28, Polysilicone-25, Polysilicone-24, Polysilicone-22, Polysilicone-19, Polysilicone-18, Polysilicone-14, Polysilicone-3, and mixtures thereof.

In an embodiment, in a composition as defined above, the at least one film-forming amino-silicone polymer is selected from Polysilicone-35, Polysilicone-33, Polysilicone-29, and mixtures thereof.

In an embodiment, a composition as defined above, wherein the at least one silane derivative of a hydrolyzed protein or peptide or amino acid is selected from Hydrolyzed Collagen PG-Propyl Methylsilanediol, Hydrolyzed Keratin PG-Propyl Methylsilanediol, Hydrolyzed Sesame Protein PG-Propyl Methylsilanediol, Hydrolyzed Silk PG-Propyl Methylsilanediol, Hydrolyzed Soy Protein PG-Propyl Methylsilanediol, Hydrolyzed Wheat Protein PG-Propyl Methylsilanediol, Hydrolyzed Vegetable Protein PG-Propyl Silanetriol, Cystine Bis-PG-Propyl Silanetriol, and mixtures thereof.

In an embodiment, a composition as defined above, the at least one silane derivative of a hydrolyzed protein or peptide or amino acid is selected from Hydrolyzed Collagen PG-Propyl Methylsilanediol, Hydrolyzed Keratin PG-Propyl Methylsilanediol, Hydrolyzed Sesame Protein PG-Propyl Methylsilanediol, Hydrolyzed Silk PG-Propyl Methylsilanediol, Hydrolyzed Soy Protein PG-Propyl Methylsilanediol, Hydrolyzed Wheat Protein PG-Propyl Methylsilanediol, and mixtures thereof.

In an embodiment disclosed herein, in the composition as defined above, the total amount of the at least one film-forming amino-silicone polymer is in the range of about 0.05 to about 10 wt.%, based on the total weight of the composition.

In an embodiment disclosed herein, in the composition as defined above, the total amount of the at least one

silane derivative of a hydrolyzed protein or peptide or amino acid is in the range of about 0.05 to about 10 wt.%, based on the total weight of the composition.

In an embodiment, a composition as defined above comprises in a cosmetically acceptable medium, based on the total weight of the composition:

a) about 0.05-10 wt.% of at least one film-forming amino-silicone polymer; and
b) about 0.05-10 wt.% of at least one silane derivative of a hydrolyzed protein or peptide or amino acid.

In an embodiment, a composition as defined above comprises, in a cosmetically acceptable medium, based on the total weight of the composition:

a) about 0.08-5 wt.% of at least one film-forming amino-silicone polymer; and
b) about 0.1-5 wt.% of at least one silane derivative of a hydrolyzed protein or peptide or amino acid.

In an embodiment, a composition as defined above comprises, in a cosmetically acceptable medium, based on the total weight of the composition:

a) about 0.05-10 wt.% of at least one film-forming amino-silicone polymer selected from Polysilicone-35, Polysilicone-33, Polysilicone-29, Polysilicone-28, Polysilicone-25, Polysilicone-24, Polysilicone-22, Polysilicone-19, Polysilicone-18, Polysilicone-14, Polysilicone-3, and mixtures thereof; and
b) about 0.05-10 wt.% of at least one silane derivative of a hydrolyzed protein or peptide or amino acid selected from Hydrolyzed Collagen PG-Propyl Methylsilanediol, Hydrolyzed Keratin PG-Propyl Methylsilanediol, Hydrolyzed Sesame Protein PG-Propyl Methylsilanediol, Hydrolyzed Silk PG-Propyl Methylsilanediol, Hydrolyzed Soy Protein PG-Propyl Methylsilanediol, Hydrolyzed Wheat Protein PG-Propyl Methylsilanediol, Hydrolyzed Vegetable Protein PG-Propyl Silanetriol, Cystine Bis-PG-Propyl Silanetriol, and mixtures thereof.

In an embodiment, a composition as defined above comprises, in a cosmetically acceptable medium, based on the total weight of the composition:

a) about 0.05-10 wt.% of at least one film-forming amino-silicone polymer selected from Polysilicone-35, Polysilicone-33, Polysilicone-29, and mixtures thereof; and
b) about 0.05-10 wt.% of at least one silane derivative of a hydrolyzed protein or peptide or amino acid selected from Hydrolyzed Collagen PG-Propyl Methylsilanediol, Hydrolyzed Keratin PG-Propyl Methylsilanediol, Hydrolyzed Sesame Protein PG-Propyl Methylsilanediol, Hydrolyzed Silk PG-Propyl Methylsilanediol, Hydrolyzed Soy Protein PG-Propyl Methylsilanediol, Hydrolyzed Wheat Protein PG-Propyl Methylsilanediol, and mixtures thereof.

In an embodiment, a composition as defined above comprises, in a cosmetically acceptable medium, based on the total weight of the composition:

a) about 0.08-5 wt.% of at least one film-forming amino-silicone polymer selected from Polysilicone-35, Polysilicone-33, Polysilicone-29, and mixtures thereof; and
b) about 0.1-5 wt.% of at least one silane derivative of a hydrolyzed protein or peptide or amino acid selected from Hydrolyzed Collagen PG-Propyl Methylsilanediol, Hydrolyzed Keratin PG-Propyl Methylsilanediol, Hydrolyzed Sesame Protein PG-Propyl Methylsilanediol, Hydrolyzed Silk PG-Propyl Methylsilanediol, Hydrolyzed Soy Protein PG-Propyl Methylsilanediol, Hydrolyzed Wheat Protein PG-Propyl Methylsilanediol, and mixtures thereof.

In an embodiment, a process for treating keratin fibers comprises the steps of:

i) applying to the keratin fibers a composition as defined above;
ii) applying heat to the keratin fibers treated in step (i).

In an embodiment, the use of a composition as defined above to protect keratin fibers from heat damage is disclosed.

In an embodiment, the use of a composition as defined above as an anti-frizz agent for keratin fibers is disclosed.

## DETAILED DESCRIPTION

Embodiments of the present disclosure are discussed in detail below. In describing embodiments, specific

terminology is employed for the sake of clarity. However, the disclosure is not intended to be limited to the specific terminology so selected. While specific exemplary embodiments are discussed, it should be understood that this is done for illustration purposes only. A person skilled in the relevant art will recognize that other components and configurations can be used without parting from the spirit and scope of the disclosure. While a number of embodiments and features are described herein, it is to be understood that the various features of the disclosure and aspects of embodiments, even if described separately, may be combined unless mutually exclusive or contrary to the specific description. All references cited herein are incorporated by reference as if each had been individually incorporated.

The terms "about" or "approximately," as used herein, shall generally mean within 10 percent of a given value. Other than in the operating examples, or where otherwise indicated, all numbers expressing quantities of ingredients and/or reaction conditions are to be understood as being modified in all instances by the term "about".

The term "keratin fibers," as used herein, refers to a family of proteins that occur in the vertebrates and exhibit a protective function. In an embodiment, keratin fibers refer to human keratin fibers. In another embodiment, keratin fibers refer to human hair, such as human hair that grows on the scalp.

The singular form "a," "an" and "the," as used herein, include plural references unless the context clearly dictates otherwise. For example, the term "a peptide" includes a plurality of peptides, including mixtures thereof.

The term "comprising" refers to optional compatible components/steps that can be used provided that the important ingredients/steps are present.

The term "comprising" thus encompasses and includes the more restrictive terms "consisting of' and "consisting essentially of".

The term "film-forming," as used herein, refers to any compound that leaves a pliable, cohesive, and continuous covering over the hair when applied to the surface of the hair.

The term "amino-silicone," as used herein, refers to silicone functionalized with at least one amino group.

The term "polymer," as used herein, comprises copolymers (including terpolymers) and homopolymers.

The term "silane," as used herein, refers to any compound with four non-hydrogen substituents on silicon, including an organosilicon compound.

The term "hydrolyzed protein," as used herein, refers to the product of the hydrolysis of homogeneous or heterogeneous proteins, or their respective components, derivatives or combinations thereof, from sources including, but not limited to, plants and their respective components, seeds, animal bones, connective tissue, animal keratin, bovine and porcine collagen, human hair, wool, silk, elastin, reticulin, milk, egg, wheat, corn, soy, oats, casein, albumin, or any collagenous or keratin substance, or derivatives thereof.

The term "peptide" refers to two or more amino acids joined to each other by peptide bonds or modified peptide bonds.

The term "amino acid" refers to the basic chemical structural unit of a protein or a peptide.

The term "frizzy hair" refers to hair that does not align with the surrounding hairs, but stands up or curls independently, creating a fuzzy or irregular texture. It can be caused by genetics, hair damage, or humidity.

The term "anti-frizz agent" refers to a cosmetic ingredient or composition that reduces the amount and/or severity of frizzy hair.

## Cosmetically acceptable medium

The cosmetically acceptable medium for the composition for treating keratin fibers may be an aqueous medium including water and may include cosmetically acceptable organic solvents including alcohols such as ethyl alcohol, isopropyl alcohol, benzyl alcohol and phenylethyl alcohol, glycols or glycol ethers such as, for example, monomethyl, monoethyl and monobutyl ethers of ethylene glycol, propylene glycol or its ethers such as, for example, monomethyl ether of propylene glycol, butylene glycol, pentylene glycol, dipropylene glycol as well as the alkyl ethers of diethylene glycol such as, for example, monoethyl ether or monobutyl ether of diethylene glycol.

Water may be present in the range of about 30 to about 95 wt. %, or about 40 to about 90 wt. %, or about 45 to about 85 wt.%, based on the total weight of the composition. One or more organic solvents may be present in concentrations of between about 0.5 and 20 wt. %, or about 2 and 15 wt.%, based on the total weight of the composition.

## The film-forming amino-silicone polymer

The film-forming amino-silicone polymer (a) may be selected from Polysilicone-35, Polysilicone-33, Polysilicone-29, Polysilicone-28, Polysilicone-25, Polysilicone-24, Polysilicone-22, Polysilicone-19, Polysilicone-18, Polysilicone-14, Polysilicone-3, and mixtures thereof.

According to the Personal Care Products Council (PCPC) On-Line INFOBASE, Polysilicone-35 is a siloxane polymer that conforms generally to the following formula:

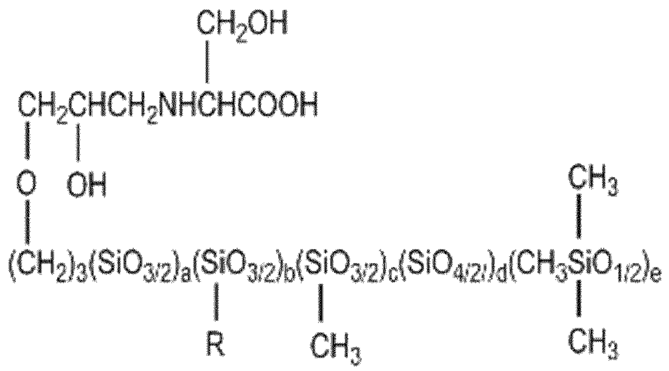

where R represents a $C_{6-10}$ alkyl chain.

According to the PCPC On-Line INFOBASE, Polysilicone-33 is a polysilsesquioxane that also contains dimethyl siloxane groups. It is produced by the hydrolysis and condensation of dimethyldimethoxysilane, Methyltrimethoxysilane, propyltrimethoxysilane, Aminopropyl Triethoxysilane, octyltriethoxysilane, and phenyltrimethoxysilane.

According to the PCPC On-Line INFOBASE, Polysilicone-29 is a complex silicone polymer formed by the reaction between a glycidoxypropyl-terminated dimethyl siloxane polymer, PEG-13 diglycidyl ether, diethylaminopropylamine, and aminopropyltriisopropoxysilane.

According to the PCPC On-Line INFOBASE, Polysilicone-28 is a complex copolymer made by reacting Aminopropyl Dimethicone with the thiolactone of acetylhomocysteine. The mercapto-modified dimethicone produced by this reaction is further copolymerized with a mixture of Dimethylacrylamide and t-Butylacrylamide.

According to the PCPC On-Line INFOBASE, Polysilicone-25 is a polymer obtained by reacting Glycidoxy Dimethicone with Stearic Acid, Oleic Acid, N-methylglucamine, and Diethanolamine.

According to the PCPC On-Line INFOBASE, Polysilicone-24 is the silicone polymer that conforms generally to the following formula:

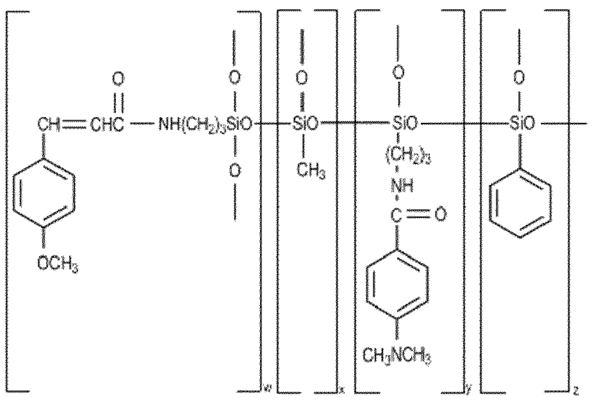

According to the PCPC On-Line INFOBASE, Polysilicone-22 is a complex siloxane polymer made by reacting Hydrogen Dimethicone with Dodecene and then crosslinking with Bis-Vinyldimethicone. The resulting material is further treated with Methyltrimethoxysilane and aminopropyl trimethoxysilane to form a resinous coating over the crosslinked siloxane.

According to the PCPC On-Line INFOBASE, Polysilicone-19 is the silicone polymer produced by the reaction of Glycidoxy Dimethicone with 4-methoxycinnamic acid, Cocamidopropyl Dimethylamine, and Lactic Acid.

According to the PCPC On-Line INFOBASE, Polysilicone-18 is the polymer prepared by reacting Ethanolamine, glycidoxy-terminated PPG-7, and glycidoxypropyl-terminated dimethicone.

According to the PCPC On-Line INFOBASE, Polysilicone-14 is the siloxane polymer that conforms generally to the following formula:

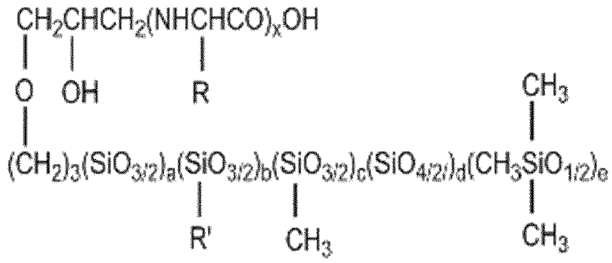

where R represents a hydrolyzed silk moiety and R' represents a C6-10 alkyl chain.

According to the PCPC On-Line INFOBASE, Polysilicone-3 is prepared from an alkylthiosulfate substituted N-acetylmethionyl silanol by hydrolysis in the presence of Dextran. It conforms to the following formula:

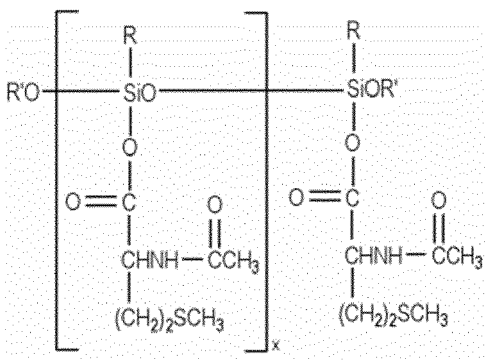

where R is $(CH_2)_3OCH_2CHOHCH_2S_2O_3Na$ and R' is Dextran.

Dextran is a high molecular weight glucose polymer produced by the action of the bacteria, Leuconostoc mesenteroides, on a sucrose substrate.

In an embodiment, the film-forming amino-silicone polymer is selected from Polysilicone-35, Polysilicone-33, Polysilicone-29, and mixtures thereof.

In one embodiment, the film-forming amino-silicone polymer comprises Polysilicone-29.

A suitable film-forming amino-silicone polymer for use in the present disclosure is available under the trademark Silsoft(R) CLX-E by the company Momentive Performance Materials. Silsoft(R) CLX-E has as the INCI (International Nomenclature Cosmetic Ingredient) name "Dipropylene glycol and Polysilicone-29".

In the composition for treating keratin fibers, the total amount of (a), the film-forming amino-silicone polymer, may be in the range of about 0.05 to 10 wt.%, or about 0.08 to about 5 wt.%, or about 0.1 to 3 wt.%, or about 0.3 to about 2 wt.%, or about 0.4 to about 1.5 wt.% based on the total weight of the composition.

## The silane derivative of a hydrolyzed protein or peptide or amino acid

The compositions of the present disclosure may be, but are not limited to, hydrolyzed proteins produced from naturally occurring proteins. Synthetic proteins, peptides, or amino acids as well as naturally occurring proteins, peptides or amino acids or mixtures of naturally occurring and synthetic proteins and/or peptides and/or amino acids may also be used. Hydrolyzed protein prepared from various proteins, their respective components, and derivatives may be combined and used in the composition of the present disclosure. Moreover, a hydrolyzed protein may be supplemented by the addition of one or more natural or synthetic peptides or amino acids.

Methods for producing hydrolyzed proteins from the abovementioned protein sources include, but are not limited to: 1) acid hydrolysis; 2) alkali hydrolysis; and 3) enzyme hydrolysis using a suitable protease.

These methods, along with several others, for preparing hydrolyzed proteins are well known in the art. Further, hydrolyzed proteins suitable for the compositions of the present disclosure are commercially available.

Hydrolyzed proteins may have a number average molecular weight from about two hundred to several hundred thousand depending on the nature of the protein and/or the extent of hydrolysis. The number average molecular weight of the hydrolyzed proteins suitable for the present disclosure is from about 100 to 100,000, or from about 100 to 20,000, or from about 100 to 10,000, or from about 100 to 5,000.

The silane derivative of a hydrolyzed protein or peptide or amino acid may be formed by covalently attaching an organofunctional silicone/silane compound to the terminal or side chain amino group of the hydrolyzed protein or

peptide or amino acid by an amino reactive group such as epoxy, esters, haloalkyl and the like.

The silane derivative of a hydrolyzed protein or peptide or amino acid may further comprise cross-linked groups. Cross-linking involves the formation of functional siloxane links between two polymer backbone chains and occurs through a mechanism in which the terminal silanol (Si-OH) of one polymer condenses with a terminal silanol of another polymer forming a siloxane (Si-O-Si) bond and water.

In an embodiment, the silane derivative of a hydrolyzed protein or peptide or amino acid is selected from Hydrolyzed Collagen PG-Propyl Methylsilanediol, Hydrolyzed Keratin PG-Propyl Methylsilanediol, Hydrolyzed Sesame Protein PG-Propyl Methylsilanediol, Hydrolyzed Silk PG-Propyl Methylsilanediol, Hydrolyzed Soy Protein PG-Propyl Methylsilanediol, Hydrolyzed Wheat Protein PG-Propyl Methylsilanediol, Hydrolyzed Vegetable Protein PG-Propyl Silanetriol, Cystine Bis-PG-Propyl Silanetriol, and mixtures thereof.

In an embodiment, the silane derivative of a hydrolyzed protein or peptide or amino acid is selected from Hydrolyzed Collagen PG-Propyl Methylsilanediol, Hydrolyzed Keratin PG-Propyl Methylsilanediol, Hydrolyzed Sesame Protein PG-Propyl Methylsilanediol, Hydrolyzed Silk PG-Propyl Methylsilanediol, Hydrolyzed Soy Protein PG-Propyl Methylsilanediol, Hydrolyzed Wheat Protein PG-Propyl Methylsilanediol, and mixtures thereof.

In an embodiment, the silane derivative of a hydrolyzed protein or peptide or amino acid is selected from Hydrolyzed Keratin PG-Propyl Methylsilanediol, Hydrolyzed Sesame Protein PG-Propyl Methylsilanediol, Hydrolyzed Silk PG-Propyl Methylsilanediol, Hydrolyzed Soy Protein PG-Propyl Methylsilanediol, Hydrolyzed Wheat Protein PG-Propyl Methylsilanediol, and mixtures thereof.

In one embodiment, the silane derivative of a hydrolyzed protein or peptide or amino acid is selected from Hydrolyzed Sesame Protein PG-Propyl Methylsilanediol, Hydrolyzed Silk PG-Propyl Methylsilanediol, Hydrolyzed Soy Protein PG-Propyl Methylsilanediol, Hydrolyzed Wheat Protein PG-Propyl Methylsilanediol, and mixtures thereof.

In the composition for treating keratin fibers, the total amount of (b), the silane derivative of a hydrolyzed protein or peptide or amino acid, may be in the range of about 0.05 to 10 wt.%, or about 0.1 to about 5 wt.%, or about 0.15 to 3 wt.% or about 0.2 to about 1 wt.%, based on the total weight of the composition.

## The compositions for treating keratin fibers

In an embodiment, the composition for treating keratin fibers is a cosmetic composition. The term "cosmetic composition" as used herein, refers to any substance or mixture intended to be placed in contact with the external parts of the human body (epidermis, hair system, nails, lips and external genital organs) or with the teeth and the mucous membranes of the oral cavity with a view exclusively or mainly to cleaning them, perfuming them, changing their appearance, protecting them, keeping them in good condition or correcting body odour.

In another embodiment, the composition for treating keratin fibers is a cosmetic composition for treating human hair.

The composition for treating keratin fibers may comprise, in a cosmetically acceptable medium: (a) at least one film-forming amino-silicone polymer; and (b) at least one silane derivative of a hydrolyzed protein or peptide or amino acid.

The film-forming amino-silicone polymer (a), may be present at a concentration ranging from about 0.05 to about 10 wt.%, or about 0.08 to about 5 wt.%, or about 0.1 to about 3 wt.% or about 0.3 to about 2 wt.%, or about 0.4 to about 1.5 wt.% based on the total weight of the composition.

The silane derivative of a hydrolyzed protein or peptide or amino acid (b) may be present at a concentration ranging from about 0.05 to about 10 wt.%, or about 0.1 to about 5 wt.%, or about 0.15 to about 3 wt.% or about 0.2 to about 1 wt.%, based on the total weight of the composition.

In an embodiment, the composition for treating keratin fibers comprises, in a cosmetically acceptable medium, based on the total weight of the composition:

a) about 0.05-10 wt.% of at least one film-forming amino-silicone polymer; and
b) about 0.05-10 wt.% of at least one silane derivative of a hydrolyzed protein or peptide or amino acid.

In an embodiment, the composition for treating keratin fibers comprises, in a cosmetically acceptable medium, based on the total weight of the composition:

a) about 0.08-5 wt.% of at least one film-forming amino-silicone polymer; and
b) about 0.1-5 wt.% of at least one silane derivative of a hydrolyzed protein or peptide or amino acid.

In an embodiment, the composition for treating keratin fibers comprises, in a cosmetically acceptable medium, based on the total weight of the composition:

a) about 0.05-10 wt.% of at least one film-forming amino-silicone polymer selected from Polysilicone-35, Polysilicone-33, Polysilicone-29, Polysilicone-28, Polysilicone-25, Polysilicone-24, Polysilicone-22, Polysilicone-19, Polysilicone-18, Polysilicone-14, Polysilicone-3, and mixtures thereof; and
b) about 0.05-10 wt.% of at least one silane derivative of a hydrolyzed protein or peptide or amino acid selected from Hydrolyzed Collagen PG-Propyl Methylsilanediol, Hydrolyzed Keratin PG-Propyl Methylsilanediol, Hydrolyzed Sesame Protein PG-Propyl Methylsilanediol, Hydrolyzed Silk PG-Propyl Methylsilanediol, Hydrolyzed Soy Protein PG-Propyl Methylsilanediol, Hydrolyzed Wheat Protein PG-Propyl Methylsilanediol, Hydrolyzed Vegetable Protein PG-Propyl Silanetriol, Cystine Bis-PG-Propyl Silanetriol, and mixtures thereof.

In an embodiment, the composition for treating keratin fibers comprises, in a cosmetically acceptable medium, based on the total weight of the composition:

a) about 0.08-5 wt.% of at least one film-forming amino-silicone polymer selected from Polysilicone-35, Polysilicone-33, Polysilicone-29, Polysilicone-28, Polysilicone-25, Polysilicone-24, Polysilicone-22, Polysilicone-19, Polysilicone-18, Polysilicone-14, Polysilicone-3, and mixtures thereof; and
b) about 0.1-5 wt.% of at least one silane derivative of a hydrolyzed protein or peptide or amino acid selected from Hydrolyzed Collagen PG-Propyl Methylsilanediol, Hydrolyzed Keratin PG-Propyl Methylsilanediol, Hydrolyzed Sesame Protein PG-Propyl Methylsilanediol, Hydrolyzed Silk PG-Propyl Methylsilanediol, Hydrolyzed Soy Protein PG-Propyl Methylsilanediol, Hydrolyzed Wheat Protein PG-Propyl Methylsilanediol, Hydrolyzed Vegetable Protein PG-Propyl Silanetriol, Cystine Bis-PG-Propyl Silanetriol, and mixtures thereof.

In an embodiment, the composition for treating keratin fibers comprises, in a cosmetically acceptable medium, based on the total weight of the composition:

a) about 0.1-3 wt.% of at least one film-forming amino-silicone polymer selected from Polysilicone-35, Polysilicone-33, Polysilicone-29, Polysilicone-28, Polysilicone-25, Polysilicone-24, Polysilicone-22, Polysilicone-19, Polysilicone-18, Polysilicone-14, Polysilicone-3, and mixtures thereof; and
b) about 0.15-3 wt.% of at least one silane derivative of a hydrolyzed protein or peptide or amino acid selected from Hydrolyzed Collagen PG-Propyl Methylsilanediol, Hydrolyzed Keratin PG-Propyl Methylsilanediol, Hydrolyzed Sesame Protein PG-Propyl Methylsilanediol, Hydrolyzed Silk PG-Propyl Methylsilanediol, Hydrolyzed Soy Protein PG-Propyl Methylsilanediol, Hydrolyzed Wheat Protein PG-Propyl Methylsilanediol, Hydrolyzed Vegetable Protein PG-Propyl Silanetriol, Cystine Bis-PG-Propyl Silanetriol, and mixtures thereof.

In an embodiment, the composition for treating keratin fibers comprises, in a cosmetically acceptable medium, based on the total weight of the composition:

a) about 0.3-2 wt.% of at least one film-forming amino-silicone polymer selected from Polysilicone-35, Polysilicone-33, Polysilicone-29, Polysilicone-28, Polysilicone-25, Polysilicone-24, Polysilicone-22, Polysilicone-19, Polysilicone-18, Polysilicone-14, Polysilicone-3, and mixtures thereof; and
b) about 0.2-1 wt.% of at least one silane derivative of a hydrolyzed protein or peptide or amino acid selected from Hydrolyzed Collagen PG-Propyl Methylsilanediol, Hydrolyzed Keratin PG-Propyl Methylsilanediol, Hydrolyzed Sesame Protein PG-Propyl Methylsilanediol, Hydrolyzed Silk PG-Propyl Methylsilanediol, Hydrolyzed Soy Protein PG-Propyl Methylsilanediol, Hydrolyzed Wheat Protein PG-Propyl Methylsilanediol, Hydrolyzed Vegetable Protein PG-Propyl Silanetriol, Cystine Bis-PG-Propyl Silanetriol, and mixtures thereof.

In an embodiment, the composition for treating keratin fibers comprises, in a cosmetically acceptable medium, based on the total weight of the composition:

a) about 0.05-10 wt.% of at least one film-forming amino-silicone polymer selected from Polysilicone-35, Polysilicone-33, Polysilicone-29, and mixtures thereof; and
b) about 0.05-10 wt.% of at least one silane derivative of a hydrolyzed protein or peptide or amino acid selected from Hydrolyzed Collagen PG-Propyl Methylsilanediol, Hydrolyzed Keratin PG-Propyl Methylsilanediol, Hydrolyzed Sesame Protein PG-Propyl Methylsilanediol, Hydrolyzed Silk PG-Propyl Methylsilanediol, Hydrolyzed Soy Protein PG-Propyl Methylsilanediol, Hydrolyzed Wheat Protein PG-Propyl Methylsilanediol, and mixtures thereof.

In an embodiment, the composition for treating keratin fibers comprises, in a cosmetically acceptable medium, based on the total weight of the composition:

a) about 0.08-5 wt.% of at least one film-forming amino-silicone polymer selected from Polysilicone-35, Polysilicone-33, Polysilicone-29, and mixtures thereof; and
b) about 0.1-5 wt.% of at least one silane derivative of a hydrolyzed protein or peptide or amino acid selected from Hydrolyzed Collagen PG-Propyl Methylsilanediol, Hydrolyzed Keratin PG-Propyl Methylsilanediol, Hydrolyzed Sesame Protein PG-Propyl Methylsilanediol, Hydrolyzed Silk PG-Propyl Methylsilanediol, Hydrolyzed Soy Protein PG-Propyl Methylsilanediol, Hydrolyzed Wheat Protein PG-Propyl Methylsilanediol, and mixtures thereof.

In an embodiment, the composition for treating keratin fibers comprises, in a cosmetically acceptable medium, based on the total weight of the composition:

a) about 0.1-3 wt.% of at least one film-forming amino-silicone polymer selected from Polysilicone-35, Polysilicone-33, Polysilicone-29, and mixtures thereof; and
b) about 0.15-3 wt.% of at least one silane derivative of a hydrolyzed protein or peptide or amino acid selected from Hydrolyzed Collagen PG-Propyl Methylsilanediol, Hydrolyzed Keratin PG-Propyl Methylsilanediol, Hydrolyzed Sesame Protein PG-Propyl Methylsilanediol, Hydrolyzed Silk PG-Propyl Methylsilanediol, Hydrolyzed Soy Protein PG-Propyl Methylsilanediol, Hydrolyzed Wheat Protein PG-Propyl Methylsilanediol, and mixtures thereof.

In an embodiment, the composition for treating keratin fibers comprises, in a cosmetically acceptable medium, based on the total weight of the composition:

a) about 0.3-2 wt.% of at least one film-forming amino-silicone polymer selected from Polysilicone-35, Polysilicone-33, Polysilicone-29, and mixtures thereof; and
b) about 0.2-1 wt.% of at least one silane derivative of a hydrolyzed protein or peptide or amino acid selected from Hydrolyzed Collagen PG-Propyl Methylsilanediol, Hydrolyzed Keratin PG-Propyl Methylsilanediol, Hydrolyzed Sesame Protein PG-Propyl Methylsilanediol, Hydrolyzed Silk PG-Propyl Methylsilanediol, Hydrolyzed Soy Protein PG-Propyl Methylsilanediol, Hydrolyzed Wheat Protein PG-Propyl Methylsilanediol, and mixtures thereof.

In an embodiment, the composition for treating keratin fibers comprises, in a cosmetically acceptable medium, based on the total weight of the composition:

a) about 0.05-10 wt.% of at least one film-forming amino-silicone polymer selected from Polysilicone-35, Polysilicone-33, Polysilicone-29, and mixtures thereof; and
b) about 0.05-10 wt.% of at least one silane derivative of a hydrolyzed protein or peptide or amino acid selected from Hydrolyzed Sesame Protein PG-Propyl Methylsilanediol, Hydrolyzed Silk PG-Propyl Methylsilanediol, Hydrolyzed Soy Protein PG-Propyl Methylsilanediol, Hydrolyzed Wheat Protein PG-Propyl Methylsilanediol, and mixtures thereof.

In an embodiment, the composition for treating keratin fibers comprises, in a cosmetically acceptable medium, based on the total weight of the composition:

a) about 0.08-5 wt.% of at least one film-forming amino-silicone polymer selected from Polysilicone-35, Polysilicone-33, Polysilicone-29, and mixtures thereof; and
b) about 0.1-5 wt.% of at least one silane derivative of a hydrolyzed protein or peptide or amino acid selected from Hydrolyzed Sesame Protein PG-Propyl Methylsilanediol, Hydrolyzed Silk PG-Propyl Methylsilanediol, Hydrolyzed Soy Protein PG-Propyl Methylsilanediol, Hydrolyzed Wheat Protein PG-Propyl Methylsilanediol, and mixtures thereof.

In an embodiment, the composition for treating keratin fibers comprises, in a cosmetically acceptable medium, based on the total weight of the composition:

a) about 0.1-3 wt.% of at least one film-forming amino-silicone polymer selected from Polysilicone-35, Polysilicone-33, Polysilicone-29, and mixtures thereof; and
b) about 0.15-3 wt.% of at least one silane derivative of a hydrolyzed protein or peptide or amino acid selected from Hydrolyzed Sesame Protein PG-Propyl Methylsilanediol, Hydrolyzed Silk PG-Propyl Methylsilanediol, Hydrolyzed Soy Protein PG-Propyl Methylsilanediol, Hydrolyzed Wheat Protein PG-Propyl Methylsilanediol, and mixtures thereof.

In an embodiment, the composition for treating keratin fibers comprises, in a cosmetically acceptable medium, based on the total weight of the composition:

a) about 0.3-2 wt.% of at least one film-forming amino-silicone polymer selected from Polysilicone-35, Polysilicone-33, Polysilicone-29, and mixtures thereof; and
b) about 0.2-1 wt.% of at least one silane derivative of a hydrolyzed protein or peptide or amino acid selected from Hydrolyzed Sesame Protein PG-Propyl Methylsilanediol, Hydrolyzed Silk PG-Propyl Methylsilanediol, Hydrolyzed Soy Protein PG-Propyl Methylsilanediol, Hydrolyzed Wheat Protein PG-Propyl Methylsilanediol, and mixtures thereof.

In an embodiment, the composition for treating keratin fibers comprises, in a cosmetically acceptable medium, based on the total weight of the composition:

a) about 0.05-10 wt.% of Polysilicone-29 and optionally one or more additional film-forming amino-silicone polymers, and mixtures thereof; and
b) about 0.05-10 wt.% of at least one silane derivative of a hydrolyzed protein or peptide or amino acid selected from Hydrolyzed Keratin PG-Propyl Methylsilanediol, Hydrolyzed Sesame Protein PG-Propyl Methylsilanediol, Hydrolyzed Silk PG-Propyl Methylsilanediol, Hydrolyzed Soy Protein PG-Propyl Methylsilanediol, Hydrolyzed Wheat Protein PG-Propyl Methylsilanediol, and mixtures thereof.

In an embodiment, in the composition for treating keratin fibers, the weight ratio between (a) the film-forming amino-silicone polymer and (b) the at least one silane derivative of a hydrolyzed protein or peptide or amino acid ranges from about 0.1:1 to about 1:0.1, or from about 0.2:1 to about 1:0.5, or from about 0.25:1 to about 1:1.

Other Components

The compositions for treating keratin fibers may contain a variety of other components to enhance the beneficial properties of these compositions, as further described herein.

1. Thickening Agents

In an embodiment, the compositions for treating keratin fibers may contain one or more agents that will provide a thickening, or viscosity increasing effect, to the compositions. Suitable thickening agents include, but are not limited to, anionic, synthetic polymers, cationic, synthetic polymers, naturally occurring thickeners, such as nonionic guar gums, scleroglucan gums or xanthan gums, gum arabic, gum ghatti, karaya gum, tragacanth gum, carrageenan gum, agar-agar, locust bean gum, pectins, alginates, starch fractions, and derivatives such as amylose, amylopectin, and dextrins, as well as cellulose derivatives such as, for example, methylcellulose, carboxyalkylcelluloses, and hydroxyalkylcelluloses (such as hydroxyethylcellulose, hydroxyethyl ethylcellulose, hydroxypropylcellulose or hydroxypropyl methylcellulose), nonionic, fully synthetic polymers, such as polyvinyl alcohol or polyvinylpyrrolidinone; as well as inorganic thickeners, such as phyllosilicates, for example, bentonite, or smectites, such as montmorillonite or hectorite. Thickening agents may be included at from about 0.001-20 wt.%, or about 0.005-15 wt.%, or about 0.01-12 wt.%, based on the total weight of the composition.

2. Preservatives

"Preservatives", as used herein, are ingredients which prevent or retard microbial growth and thus help protect cosmetic products from spoilage. Preservatives may also protect the product from inadvertent contamination by the consumer during use. Suitable preservatives include, but are not limited to, methyl, ethyl, and propyl paraben, hydantoins, and the like. Preservatives may be included at about 0.0001-8 wt.%, or about 0.0005-7 wt.%, or about 0.001-5 wt.%, based on the total weight of the composition.

3. Chelating Agents

The compositions for treating keratin fibers may contain about 0.0001-5 wt.%, or 0.0005-3 wt.%, or 0.001-2 wt.%, based on the total weight of the composition, of one or more chelating agents. Chelating agents may be capable of complexing with and inactivating metallic ions in order to help prevent their adverse effects on the stability or effects of the composition. Suitable chelating agents include, but are not limited to, sodium citrate; nitrogen-containing polycarboxylic acids, such as Ethylenediaminetetraacetate (EDTA), and calcium, sodium, potassium or triethanolamine derivatives thereof, Hydroxyethyl Ethylenediamine Triacetic Acid (HEDTA), Ethylenediamine-N,N'-disuccinic acid (EDDS); and phosphonates, such as 1-hydroxyethane-1,1-diphosphonate (HEDP), and/or ethylenediamine tetramethylene phosphonate (EDTMP), and/or diethylenetriamine pentamethylene phosphonate (DTPMP), or sodium salts thereof.

4. pH Adjusters

In embodiments, acids, bases or buffering systems may be added to adjust the pH of the compositions for treating keratin fibers to the desired pH range. Suitable acids include hydrochloric acid, phosphoric acid, citric acid, and the like. Suitable bases include sodium or potassium hydroxide, monoethanolamine, diethanolamine, triethanolamine, aminomethyl propanol, and the like. pH adjusters may be included at from about 0.00001-8 wt.%, or about 0.00005-6 wt.%, or about 0 0001-5 wt.%, based on the total weight of the composition.

5. Pigments

The compositions for treating keratin fibers may contain inorganic pigments. Suitable inorganic pigments include, but are not limited to, those having a color index number as listed in the CTFA dictionary, 10th edition, 2004, hereby incorporated by reference.

6. Direct dyes

The compositions for treating keratin fibers may contain direct dyes. Direct dye, also called substantive dye, is a dye that adheres to its substrate by non-ionic forces, e.g., without help from other chemicals. For purposes of this disclosure dyes include direct dyes selected from anionic, cationic and non-ionic nitro dyes.
Examples of anionic direct dyes include, but are not limited to, Bromophenol Blue, Tetrabromophenol Blue, Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 5, FD&C Yellow No. 6, FD&C Blue 1, Food Black 1, Food Black 2, Disperse Black 9 and Disperse Violet 1, and their alkali metal salts such as sodium or potassium.
Examples of cationic dyes include, but are not limited to, Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Red 2, Basic Red 12, Basic Red 22, Basic Red 76, Basic Violet 1, Basic Violet 2, Basic Violet 3, Basic Violet 10, Basic Violet 14, Basic Yellow 57, Basic red 51, Basic Yellow 87 and Basic Orange 31.
Examples of non-ionic nitro dyes (HC dyes) include, but are not limited to, HC Blue No. 2, HC Blue No. 4, HC Blue No. 5, HC Blue No. 6, HC Blue No. 7, HC Blue No. 8, HC Blue No. 9, HC Blue No. 10, HC Blue No. 11, HC Blue No. 12, HC Blue No. 13, HC Blue No. 15, Blue No. 16, HC Blue No. 18, HC Brown No. 1, HC Brown No. 2, HC Green No. 1, HC Orange No. 1, HC Orange No. 2, HC Orange No. 3, HC Orange No. 5, HC Red BN, HC Red No. 1, HC Red No. 3, HC Red No. 7, HC Red No. 8, HC Red No. 9, HC Red No. 10, HC Red No. 11, HC Red No. 13, HC Red No. 14, HC Red No. 18, HC Red No. 54, HC Violet BS, HC Violet No. 1, HC Violet No. 2, HC Yellow No. 2, HC Yellow No. 4, HC Yellow No. 5, HC Yellow No. 6, HC Yellow No. 7, HC Yellow No. 8, HC Yellow No. 9, HC Yellow No. 10, HC Yellow No. 11, HC Yellow No. 12, HC Yellow No. 13, HC Yellow No. 14, HC Yellow No. 15, 2-Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

7. Fragrance or perfumes

The compositions for treating keratin fibers may include one or more fragrances or perfumes. For purposes of this disclosure, the term "fragrance" refers to any substance, natural or synthetic, used to impart an odour to a product.
For purposes of this disclosure, the term "perfume" refers to any mixture of fragrant essential oils and aroma compounds, fixatives, and solvents used to give the human body, objects, and living spaces a lasting and pleasant smell.

8. Anionic surfactants

The compositions for treating keratin fibers may include one or more anionic surfactants. Non-limiting examples of anionic surfactants include, but are not limited to, alkyl ether carboxylates, alkyl sulfates, alkyl ether sulfates, amide ether sulfates, alkyl glyceride sulfates, olefin sulfonates, alkyl-aryl sulfonates, sulfosuccinates, sulfo fatty acid esters, fatty acid isethionates, fatty acid taurides, phosphate esters, acyl glutamates, acyl peptides, acyl sarcosides, and mixtures thereof.
Examples of alkyl ether carboxylates, alkyl sulfates, alkyl ether sulfates, olefin sulfonates, and mixtures thereof

include Potassium Laureth-4 Carboxylate, Sodium Laureth-4 Carboxylate, Sodium Laureth-5 Carboxylate, Sodium Laureth-6 Carboxylate, and Sodium Laureth-11 Carboxylate, Ammonium Laureth Sulfate, Monoethanolamine (MEA)-Laureth Sulfate, Monoisopropanolamine (MIPA)-Laureth Sulfate, Sodium Laureth Sulfate, and Triethanolamine (TEA)-Laureth Sulfate, Sodium C14-C16 Olefin Sulfonate, and mixtures thereof.

9. Cationic surfactants

The compositions for treating keratin fibers may include one or more cationic surfactants. Non-limiting examples of cationic surfactants include, but are not limited to, alkylimidazolines, tetra alkyl(-aryl) quaternary ammonium salts (quats), heterocyclic ammonium salts, quaternized alkyl polyglycosides, quaternized derivatives of polyalkanolamine esters (esterquats), and mixtures thereof.

Examples of C6-C24 alkyl trimethyl quaternary ammonium salts, C6-C24 alkyl dimethyl benzyl quaternary ammonium salts, C6-C24 dialkyl dimethyl quaternary ammonium salts, and mixtures thereof include Laurtrimonium bromide, Laurtrimonium chloride, Myrtrimonium bromide, Myrtrimonium chloride, Cetrimonium bromide, Cetrimonium chloride, Cetrimonium methosulfate, Steartrimonium bromide, Steartrimonium chloride, Steartrimonium methosulfate, Behentrimonium bromide, Behentrimonium chloride, Behentrimonium methosulfate, Ceteartrimonium chloride, Cocotrimonium chloride, Cocotrimonium methosulfate, Soytrimonium chloride, Octyl dodecyl trimethyl ammonium chloride, Dodecyl hexadecyl trimethyl ammonium bromide, Dodecyl hexadecyl trimethyl ammonium chloride, Benzalkonium chloride, benzyl-C10-16-alkyldimethylammonium chloride, benzyl-C12-14-alkyldimethylammonium chloride, Didecyldimonium chloride, Dilauryldimonium chloride, Distearyldimonium chloride, Behenoyl PG-Trimonium Chloride, Dioleoylethyl Hydroxyethylmonium Methosulfate, and mixtures thereof.

10. Nonionic surfactants

The compositions for treating keratin fibers may include one or more nonionic surfactants. Non-limiting examples of nonionic surfactants include, but are not limited to, polyethoxylated and/or polypropoxylated C6-C24 fatty alcohols, polyethoxylated and/or polypropoxylated fatty acids, alkylphenols, alpha-diols or alcohols having a fatty chain containing, for example, 8 to 18 carbon atoms, ethylene oxide or propylene oxide groups to range from 2 to 50; copolymers of ethylene oxide and of propylene oxide, polyethoxylated fatty amides having from 2 to 30 mol of ethylene oxide; polyglycerolated fatty amides containing on average 1 to 5, or 1.5 to 4, glycerol groups; polyethoxylated fatty amines having 2 to 30 mol of ethylene oxide; oxyethylenated fatty acid esters of sorbitan having from 2 to 30 mol of ethylene oxide; polyethoxylated castor oil, polyethoxylated hydrogenated castor oil, fatty acid esters of sucrose; fatty acid esters of polyethylene glycol; alkylpolyglycosides; N-alkylglucamine derivatives; amine oxides such as (C10-C14) alkylamine oxides or N-acylaminopropylmorpholine oxides.

11. Amphoteric or zwitterionic surfactants

The compositions for treating keratin fibers may include one or more amphoteric or zwitterionic surfactants. Non-limiting examples of amphoteric or zwitterionic surfactants include, but are not limited to, aliphatic secondary or tertiary amine derivatives in which the aliphatic radical is a linear or branched chain containing 8 to 18 carbon atoms and containing at least one water-soluble anionic group (for example carboxylate, sulphonate, sulphate, phosphate or phosphonate); (C8-C20) alkylbetaines, sulphobetaines, (C8-C20) alkylamido (C1-C6) alkylbetaines or (C8-C20) alkylamido (C1-C6) alkylsulphobetaines; amine derivatives, disodium cocoamphodiacetate, disodium lauroamphodiacetate, disodium caprylamphodiacetate, disodium capryloamphodiacetate, disodium cocoamphodipropionate, disodium lauroamphodipropionate, disodium caprylamphodipropionate, disodium capryloamphodipropionate, lauroamphodipropionic acid, and cocoamphodipropionic acid.

12. Petroleum hydrocarbons (mineral oils, paraffins and waxes)

The compositions for treating keratin fibers may include one or more petroleum hydrocarbons. For purposes of this disclosure petroleum hydrocarbons refer to mineral oils, paraffins and waxes based on petroleum. Examples include, but are not limited to, hard paraffin, liquid paraffin (Liquid Petrolatum or Paraffinum Liquidum), light liquid paraffin (Light Liquid Petrolatum or Paraffinum Perliquidium), white soft paraffin (White Petrolatum), yellow soft paraffin (Yellow Petrolatum), macrocrystalline paraffin waxes (which are mixtures that consist mainly of saturated C18-C30 hydrocarbons and smaller amounts of iso-alkanes and cycloalkanes with a molecular weight between 250 and 450 g/mol and, although they are solids at room temperature, they have low melting points, usually between 40°C and 60°C), microcrystalline paraffin waxes (which consist of C40-C55 compounds that contain, in addition to normal hydrocarbons, included in the group are iso-alkanes and naphtenes with long alkyl side-chains, the iso-alkanes forming microcrystals, the microcrystalline paraffin waxes having mean molecular weights between 500 and 800 g/mol, being solids at room temperature, and having melting points between 60°C and 90°C), or mixtures thereof.

13. C6-C24 fatty alcohols

The compositions for treating keratin fibers may include one or more C6-C24 fatty alcohols. C6-C24 fatty alcohols from vegetable fats and oils include cotton, safflower, coconut, rapeseed, linseed, palm, palm kernel, sunflower, olein, olive, olive pomace, castor oil, soy, tall oil, etc., totally or partially hydrogenated, as well as purified or synthetic fatty alcohols such as caproyl alcohol, capryl alcohol, capric alcohol, lauryl alcohol, myristyl alcohol, palmytil (cetyl) alcohol, palmitoyl alcohol, stearyl alcohol, isostearyl alcohol, 2-octyldodecanol, 2-ethylhexanoyl alcohol, oleyl alcohol, ricinoleyl alcohol, elaidyl alcohol, petroselinic alcohol, linoleyl alcohol, linolenyl alcohol, arachidyl alcohol, gadoleyl alcohol, behenyl alcohol and erucyl alcohol, or technical grade mixtures.

14. Vegetable fats and oils

The compositions for treating keratin fibers may include one or more vegetable fats and/or oils. For purposes of this disclosure, vegetable fats and oils are linear and/or branched esters, linear or branched, saturated and/or unsaturated alkanecarboxylic acids with a chain length of 1 up to 30 carbon atoms, saturated and/or unsaturated alcohols with a chain length of 1 up to 30 carbon atoms; or linear and/or branched esters of aromatic carboxylic acids, or saturated and/or unsaturated alcohols with a chain length of 1 up to 30 carbon atoms. These oils can be selected from, for example, isopropyl myristate, isopropyl palmitate, isopropyl stearate, isopropyl oleate, n-butyl stearate, n-hexyl laurate, n-decyl oleate, isooctyl stearate, isononyl stearate, isononyl isononanoate, 2-ethylhexyl laurate, 2-ethylhexyl palmitate, 2-ethylhexyl cocoate, 2-hexyldecyl stearate, 2- ethylhexyl isostearate, 2-octyldodecyl palmitate, cetyl palmitate, stearyl palmitate, oleyl palmitate, oleyl oleate, oleyl erucate, erucyl oleate, erucyl erucate, as well as synthetic, semisynthetic and natural mixtures esters, such as jojoba oil (a natural mixture of esters of monounsaturated monocarboxylic acids with a C18-C24 chain with also monounsaturated monoalcohols and with a long C18-C24 chain).

Other examples of vegetable fats and oils include ester oils such as sugar esters or diesters of C12-C24 fatty acids. The term "sugar" means compounds comprising several alcohol functional groups, with or without an aldehyde or ketone function, and which comprise at least 4 carbon atoms. These sugars may be monosaccharides, oligosaccharides or polysaccharides.

Non-limiting examples of sugars that may be used according to the present disclosure include sucrose (or saccharose), glucose, galactose, ribose, fucose, maltose, fructose, mannose, arabinose, xylose and lactose, and derivatives thereof, including for example, alkyl derivatives such as methyl derivatives, for instance methylglucose. Non-limiting examples of the sugar esters of fatty acids that may be used according to this disclosure include those from the group comprising esters or mixtures of esters of sugars described above and of linear or branched, saturated or unsaturated C12-C24 fatty acids.

The esters may be chosen from mono-, di-, tri-, tetraesters and polyesters, and mixtures thereof. These esters may be chosen from, for example, oleates, laurates, palmitates, myristates, behenates, cocoates, stearates, linoleates, linolenates, caprates and arachidonates, or mixtures thereof such as oleo-palmitate, oleo-stearate and palmito-stearate mixed esters. The sucrose, glucose or methylglucose monoesters and diesters and for example sucrose, glucose or methylglucose mono- or dioleates, stearates, behenates, oleopalmitates, linoleates, linolenates and oleostearates, constitute sugar esters or diesters of C12-C24 fatty acids that are suitable in the context of the present disclosure. One example is methylglucose dioleate.

Other suitable oils of the type of esters of saturated alkane carboxylic acids and alcohols are fatty acid methyl esters, such as C12-C24 fatty acid methyl esters from animal and vegetable fats and oils such as cotton, safflower, coconut, rapeseed, linseed, palm, palm kernel, sunflower, olein, olive, olive pomace, castor oil, soy, tall oil, etc., totally or partially hydrogenated, as well as purified or synthetic fatty acids such as caproic acid, caprylic acid, capric acid, lauric acid, myristic acid, palmitic acid (cetylic acid), palmitoleic acid, stearic acid, isostearic acid, 2-ethylhexanoic acid, oleic acid, ricinoleic acid, elaidic acid, petroselinic acid, linoleic acid, linolenic acid, arachidic acid, gadoleic acid, behenic acid and erucic acid, or mixtures thereof.

Other suitable vegetable fats and oils according to the present disclosure are fatty acid triglycerides, including for example, linear and/or branched triglycerin esters, saturated and/or unsaturated alkanecarboxylic acids with a chain length of 6 up to 24 carbon atoms, such as 10 up to 18 carbon atoms. The fatty acids esterifying the different positions of glycerin can be different, giving rise to a large amount of possible combinations, including positional combinations. The position of the different fatty acids in natural triglycerides is not random, but rather it depends on the origin of the fat. The most simple triglycerides are those constituted by a sole fatty acid.

Fatty acid triglycerides can be chosen, for example, from synthetic, semi-synthetic and natural oils, for example avocado oil, almond oil, hazelnut oil, babassu palm oil, borage oil, peanut oil, canola oil, hemp oil, milk thistle oil, safflower

oil, chufa oil, coconut oil, rapeseed oil, black cumin oil, wheat germ oil, sunflower oil, linseed oil, macadamia nut oil, corn oil, walnut oil, olive oil and its by-products such as olive pomace oil, palm oil and its fractions such as palm olein and palm stearin, evening primrose oil, rosehip oil, castor oil, rice bran oil, apricot kernel oil, cottonseed oil, pumpkinseed oil, palm kernel oil and its fractions such as palm kernel olein and palm kernel stearin, grape seed oil, sesame oil, soy oil, cocoa butter, shea butter and the like.

15. Natural waxes

The compositions for treating keratin fibers may include one or more natural waxes. Natural waxes according to the present disclosure, include but are not limited to, candelilla wax, carnauba wax, Japan wax, esparto wax, cork wax, guaruma wax, rice wax, sugar cane wax, ouricury wax, montan wax, beeswax, shellac wax, espermaceti, wool lanolin (wax), uropygial fat wax, ceresin waxes, peat waxes, ozokerite, as well as chemically modified waxes (hard waxes) for example, montan wax esters, waxes obtained by the Fischer-Tropsch process, hydrogenated jojoba waxes and synthetic waxes.

16. Silicones different from the film-forming amino-silicone polymer or the silane derivative of a hydrolyzed protein or peptide or amino acid

The compositions for treating keratin fibers may include one or more silicones other than the film-forming amino-silicone polymer or the silane derivative of a hydrolyzed protein or peptide or amino acid. Non-limiting examples of silicones different from the film-forming amino-silicone polymer or the silane derivative of a hydrolyzed protein or peptide or amino acid in the context of the present disclosure include, but are not limited to, cyclic and/or linear silicones, which can be found as monomers generally characterized by structural elements such as:

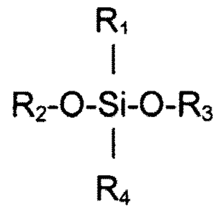

where the silicon atoms can be substituted by alkyl or aryl radicals the same or different, represented here generally by $R_1$-$R_4$ groups.

Linear silicones with siloxane units suitable according to the present disclosure are generally characterized by structural elements such as:

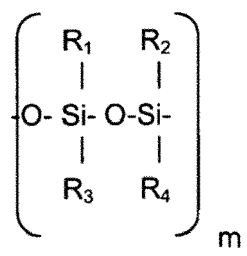

where the silicon atoms can be substituted by alkyl or aryl radicals the same or different, represented here in general by $R_1$-$R_4$ groups (meaning the number of different radicals is not limited to 4), and m can range from 2 to 200.000.

Cyclic silicones suitable according to the present disclosure are generally characterized by structural elements such as:

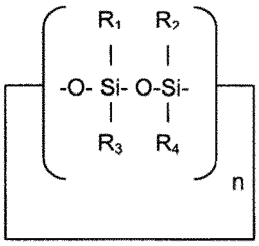

where the silicon atoms can be substituted by alkyl or aryl radicals the same or different, represented here generally by $R_1$-$R_4$ groups (meaning the number of different radicals is not limited to 4), and n can range from 3/2 to 20. Fractional values of n indicate that odd numbers of siloxane groups may be present in the ring.

Examples include a cyclic methyl siloxane having the formula $[(CH_3)_2SiO]x$ in which x is 3-6, or short chain linear methyl siloxanes having the formula $((CH_3)_2SiO[(CH_3)_2SiO]_ySi(CH_3)_3$ in which y is 0-5.

Some suitable cyclic methyl siloxanes are hexamethylcyclotrisiloxanes (D3), octamethylcyclotetrasiloxane (D4), decamethylcyclopentasiloxane (D5) (cyclomethicone) and dodecamethylcyclohexasiloxane (D6).

Some suitable short linear methyl siloxane are hexamethyldisiloxane (MM), octamethyltrisiloxane (MDM), decamethyltetrasiloxane (MD2M), dodecamethylpentasiloxane (MD3M), tetradecamethylhexasiloxane (MD4M), and hexadecamethylheptasiloxane (MD5M).

Long chain linear siloxanes such as phenyltrimethicone, bis(phenylpropyl)dimethicone, dimethicone, dimethiconol, cetyldimethicone and behenoxy dimethicone are also suitable silicones according to the present disclosure.

17. Cationic Polymers

The compositions for treating keratin fibers may include one or more cationic polymers. The term "cationic polymer" as used herein refers to a macromolecule (polymer) that contains at least one monomer bearing a quaternary ammonium group.

Examples of suitable cationic polymers include, but are not limited to, those polymers known by their International Nomenclature for Cosmetic Ingredients (INCI) name as Polyquaternium. Some examples of those are Polyquaternium-1, Polyquaternium-2, Polyquaternium-4, Polyquaternium-5, Polyquaternium-6, Polyquaternium-7, Polyquaternium-8, Polyquaternium-9, Polyquaternium-10, Polyquaternium-11, Polyquaternium-12, Polyquaternium-13, Polyquaternium-14, Polyquaternium-15, Polyquaternium-16, Polyquaternium-17, Polyquaternium-18, Polyquaternium-19, Polyquaternium-20, Polyquaternium-22, Polyquaternium-24, Polyquaternium-27, Polyquaternium-28, Polyquaternium-29, Polyquaternium-30, Polyquaternium-31, Polyquaternium-32, Polyquaternium-33, Polyquaternium-34, Polyquaternium-35, Polyquaternium-36, Polyquaternium-37, Polyquaternium-39, Polyquaternium-42, Polyquaternium-43, Polyquaternium-44, Polyquaternium-45, Polyquaternium-46, Polyquaternium-47, Polyquaternium-48, Polyquaternium-49, Polyquaternium-50, Polyquaternium-51, Polyquaternium-52, Polyquaternium-53, Polyquaternium-54, Polyquaternium-55, Polyquaternium-56, Polyquaternium-57, Polyquaternium-58, Polyquaternium-59, Polyquaternium-60, Polyquaternium-61, Polyquaternium-62, Polyquaternium-63, Polyquaternium-64, Polyquaternium-65, Polyquaternium-66, Polyquaternium-67, Polyquaternium-68, Polyquaternium-69, Polyquaternium-70, Polyquaternium-71, Polyquaternium-72, Polyquaternium-73, Polyquaternium-74, Polyquaternium-75, Polyquaternium-76, Polyquaternium-77, Polyquaternium-78, Polyquaternium-79, Polyquaternium-80, Polyquaternium-81, Polyquaternium-82, Polyquaternium-83, Polyquaternium-84, Polyquaternium-85, Polyquaternium-86, Polyquaternium-87, Polyquaternium-88, Polyquaternium-91, Polyquaternium-92, Polyquaternium-94, Polyquaternium-95, Polyquaternium-96, Polyquaternium-98, Polyquaternium-99, Polyquaternium-100, Polyquaternium-102, Polyquaternium-104, Polyquaternium-109, Polyquaternium-110, Polyquaternium-111, Polyquaternium-112, Polyquaternium-113 and Polyquaternium-114. Other suitable cationic polymers include those known by their International Nomenclature for Cosmetic Ingredients (INCI) name as Guar hydroxypropyl trimonium chloride, Polyacrylamidopropyltrimonium Chloride, Polymethacrylamidopropyltrimonium Chloride and Polymethacrylamidopropyltrimonium Methosulfate.

18. Non-ionic polymers

The compositions for treating keratin fibers may include one or more non-ionic polymers. Non-limiting examples of non-ionic polymers include, but are not limited to: nonionic guar gums and modified nonionic guar gums, which may be those modified with C1-C6 hydroxyalkyl groups; biopolysaccharide gums of microbial origin such as scleroglucan gum or xanthan gum; gums derived from plant exudates, such as gum arabic, ghatti gum, karaya gum, tragacanth gum, carrageenan, agar and carob gum; pectins; alginates; starches; hydroxy (C1-C6) alkylcelluloses and carboxy (C1-C6) alkylcelluloses; celluloses modified with groups comprising at least one fatty chain; non-limiting examples include hydroxyethylcelluloses modified with groups comprising at least one fatty chain, such as alkyl, arylalkyl or alkylaryl groups, or mixtures thereof, and wherein the alkyl groups are, for example, C8-C22; or those modified with polyalkylene glycol alkylphenyl ether groups; hydroxypropyl guars modified with groups comprising at least one C8-C22 fatty chain, copolymers of vinylpyrrolidone and of hydrophobic monomers comprising a fatty chain, such as vinylpyrrolidone/hexadecene copolymer or vinylpyrrolidone/eicosene copolymer; copolymers of C1-C6 alkyl acrylates or methacrylates and of amphiphilic monomers comprising at least one fatty chain; copolymers of hydrophilic acrylates or methacrylates and of hydrophobic monomers comprising at least one fatty chain, such as the polyethylene glycol methacrylate/lauryl methacrylate copolymer; polymers with an aminoplast ether skeleton comprising at least one fatty chain; and polyurethane polyethers comprising in their chain both hydrophilic blocks, for example of polyoxyethylenated nature, and hydrophobic blocks that may be aliphatic blocks alone and/or cycloaliphatic and/or aromatic blocks.

19. Amphoteric polymers

The compositions for treating keratin fibers may include one or more amphoteric polymers. Non-limiting examples of amphoteric polymers include, but are not limited to, amphoteric polysaccharides such as Carboxymethylchitosan or N-[(2'-Hydroxy-2',3'-dicarboxy)ethyl]chitosan; Amphoteric Urethanes; Modified Potato Starch; Methacryloyl Ethyl Betaine/Acrylates Copolymer; Acrylates/Lauryl Acrylate/Stearyl Acrylate/Ethylamine Oxide Methacrylate; and Acrylic acid/(meth)acrylamidopropyltrimethylamonium chloride/stearyl methacrylate terpolymer.

20. Anionic polymers

The compositions for treating keratin fibers may include one or more anionic polymers. Non-limiting examples of anionic polymers include, but are not limited to, those polymers known by their International Nomenclature for Cosmetic Ingredients (INCI) name as Shellac, Acrylates Copolymer, Acrylates/C1-2 Succinates/Hydroxyacrylates Copolymer, Acrylic Esters (and) Methacrylic Esters Copolymer, AMP-Acrylates/Allyl Methacrylate Copolymer, Diglycol/Cyclohexanedimethanol/Isophtalates/Sulfoisophtalates Copolymer, Diglycol/Isophtalates/Sulfoisophtalates Copolymer, Isobutylene Ethylmaleimide/Hydroxyethylmaleimide Copolymer, Glycerin and Diglycol/Cyclohexanedimethanol/Isophtalates/Sulfoisophtalates Copolymer, Methacrylate Acid/Sodium Acrylamidomethyl Propane Sulfonate Copolymer, Octylacrylamide/Acrylates/Butylaminoethylmethacrylate Copolymer, Polyurethane (and) Acrylates Copolymer, PVM/MA Copolymer, PVP/Ethyl Methacrylate/Methacrylic Acid Terpolymer, PVP/Polycarbamyl Polyglycol Ester, VA/Crotonates Copolymer, VA/Crotonates/Vinyl Neodecanoate Copolymer, VP/Acrylates/Lauryl Methacrylate Copolymer, and their cosmetically acceptable salts.

21. Other additives

The compositions for treating keratin fibers may include one or more additives other than those described above. Non-limiting examples of additives different from those listed above include, but are not limited to, Vitamins and Provitamins, such as beta-carotene, retinal, retinol, retinoic acid, biotin, panthenol, panthenyl ethyl ether, ascorbic acid, ascorbyl glucoside, ascorbyl palmitate, ascorbyl phosphate, tocopherol, tocopheryl acetate, tocopheryl glucoside, tocopheryl palmitate, and the like; hair strengthening agents such as maleic acid, hydroxypropylgluconamide (and) hydroxypropylammonium gluconate, and the like; antioxidants (ingredients employed in cosmetics to prevent or retard product spoilage from rancidity or deterioration from reaction with oxygen); moisturizers such as allantoin; natural extracts (substances or active ingredients with desirable properties that are removed from a plant, flower, alga, fungus or bacteria); UV filters (ingredients used to absorb or reflect the UV rays that are contained in sun light or in artificial light); or mixtures thereof.

In an embodiment, a composition for treating keratin fibers comprises, in a cosmetically acceptable medium:

(a) at least one film-forming amino-silicone polymer;
(b) at least one silane derivative of a hydrolyzed protein or peptide or amino acid; and
(c) at least one additional cosmetic ingredient selected from thickening agents; preservatives; chelating agents; pH adjusters; pigments; direct dyes; fragrance or perfumes; anionic surfactants; cationic surfactants; nonionic surfactants; amphoteric or zwitterionic surfactants; petroleum hydrocarbons (mineral oils, paraffins and waxes); C6-C24 fatty alcohols; vegetable fats and oils; natural waxes; silicones different from the film-forming amino-silicone polymer or the silane derivative of a hydrolyzed protein or peptide or amino acid; cationic polymers; non-ionic polymers; anionic polymers; and other additives such as vitamins and provitamins; hair strengthening agents; antioxidants; moisturizers; natural extracts; UV filters; or mixtures thereof.

Processes for treating keratin fibers

In an embodiment, a process for treating keratin fibers comprises the steps of:

i) applying to the keratin fibers a composition as defined above;
ii) applying heat to the keratin fibers treated in step (i).

In an embodiment a cosmetic process for treating keratin fibers comprises the steps of:

i) applying to the keratin fibers a composition as defined above;
ii) applying heat to the keratin fibers treated in step (i).

In an embodiment, a non-therapeutic cosmetic process for treating keratin fibers comprises the steps of:

i) applying to the keratin fibers a composition as defined above;
ii) applying heat to the keratin fibers treated in step (i).

In step i) the composition may be applied to the keratin fibers, from about 5 seconds to about 45 minutes, or from about 15 seconds to about 30 minutes, or from about 30 seconds to about 15 minutes, before step ii), i.e. before applying heat to the keratin fibers treated in step (i).
In some implementations, heat is applied to the keratin fibers just after applying to the keratin fibers a composition as defined above, i.e., step ii) is applied from about 1 to about 10 seconds after having finalized step i).
In an embodiment, the use of a composition as defined above to protect keratin fibers from heat damage is disclosed.
In an embodiment a method of protecting keratin fibers from heat damage comprises the topical application of the composition as defined above to said keratin fibers.
Protection from heat damage is obtained when the keratin fibers treated in step (i) are subjected to heat treatments with temperatures ranging from about 50°C to about 260°C, or from about 80°C to about 250°C, or from about 120°C to about 240°C, or from about 150°C to about 230°C.
In embodiments, the heat treatment may be provided by directly contacting the keratin fibers with a heat source, e.g., by heat styling of the keratin fibers. Non-limiting examples of heat styling include flat ironing and curling methods using elevated temperatures (such as, for example, setting hair in curlers and heating, and curling with a curling iron and/or hot rollers).
In embodiments, the heat treatment may be provided by heating the keratin fibers with a heat source which may not directly contact the keratin fibers. Non-limiting examples of heat sources which may not directly contact the keratin fibers include blow dryers, hood dryers, heating caps and steamers.
In an embodiment, the use of a composition as defined above as an anti-frizz agent for keratin fibers is disclosed.
In an embodiment a method of reducing frizz of keratin fibers comprises the topical application of a composition as defined above to said keratin fibers.
In an embodiment, the use of a composition as defined above as a reinforcing and repairing agent for keratin fibers, which are subjected to heat treatment with temperatures ranging from about 50°C to about 260°C, or from about 80°C to about 250°C, or from about 120°C to about 240°C, or from about 150°C to about 230°C, is disclosed.
In an embodiment a method of reinforcing and repairing keratin fibers comprises the topical application of composition as defined above to said keratin fibers and subsequently applying to the keratin fibers a heat treatment with temperatures ranging from about 50°C to about 260°C, or from about 80°C to about 250°C, or from about 120°C to about 240°C, or from about 150°C to about 230°C.
In one embodiment, the compositions for treating keratin fibers are "leave-on" compositions. The term "leave-on" as used herein refers to compositions that contain ingredients that are intended to be deposited and left on the hair for extended periods (i.e. several hours or days) until the ingredients are subsequently removed by water and/or shampooing the hair.
No particular limitation is imposed on the form of the compositions for treating keratin fibers of the present disclosure. Examples include hair sprays, setting lotions, mousses, gels, waxes, creams, pomades, gums or serums (Thomas Clausen et al., Hair Preparations, Ullmann's Encyclopedia of Industrial Chemistry, 2006).
In embodiments, the compositions are formulated as a hair spray, setting lotion or mousse.
Hair sprays may be aerosol sprays, which may be two-phase aerosols in which both a liquid and a gaseous phase are present inside the container; or pump sprays (non-aerosol sprays), which do not employ a propellant gas and the energy needed to swirl and atomize the liquid must be supplied by a pump, which is operated with a finger or through a lever.

Setting lotions may be thin, aqueous-alcohol solutions in single- or multiple-application packages.

Mousses may be thin, aqueous or aqueous-alcohol solutions in, for example, alumina cans with a propellant; or non-aerosol mousses, where the mousse is generated through use of a squeeze or pump dispenser with a riddle in the head of the pump. The liquid is mixed with air in the head of the pump and dispensed as mousse.

The pH of the compositions for treating keratin fibers of the present disclosure may be in the range of about 5.5 to about 8.5, or in the range of about 6.0 to about 8.0.

The following examples are given in order to provide a person skilled in the art with a sufficiently clear and complete explanation of the present disclosure, but should not be considered as limiting of the essential aspects of its subject, as set out in the preceding portions of this description.

## EXAMPLES

1. Preparation of the compositions

The compositions as shown in Table 1 and Table 2 were prepared by mixing and stirring until complete homogenization in a kettle. Compositions C1 to C3 (Table 1) are compositions according to the present disclosure. Compositions CC1 to CC6 (Table 2) are comparative compositions.

Table 1 - Compositions for treating keratin fibers (wt.% based on the total weight of the composition)

| Ingredients (INCI name) | Compositions for treating keratin fibers | | |
|---|---|---|---|
| | C1 | C2 | C3 |
| Hydrolyzed Wheat Protein PG-propyl Methylsilandiol | 0.0575 | 0.575 | 1.035 |
| Polysilicone-29 | 0.050 | 0.300 | 1.000 |
| Aqua (Water (Eau)) | Up to 100 | Up to 100 | Up to 100 |

Table 2 - Comparative compositions (wt.% based on the total weight of the composition)

| Ingredients (INCI name) | Comparative Compositions (CC) | | | | | |
|---|---|---|---|---|---|---|
| | CC1 | CC2 | CC3 | CC4 | CC5 | CC6 |
| Hydrolyzed Wheat Protein PG-propyl Methylsilandiol | 0.0575 | 0.575 | 1.035 | --- | --- | --- |
| Polysilicone-29 | --- | --- | --- | 0.050 | 0.300 | 1.000 |
| Aqua (Water (Eau)) | Up to 100 | Up to 100 | Up to 100 | Up to 100 | Up to 100 | Up to 100 |

2. Application of the compositions to keratin fibers

2.1. Pre-wash

Strands of virgin dark Caucasian hair were selected, each one approximately 22 cm long.
24 hours prior to the treatment, the strands were washed with a shampoo and rinsed under tap water according to the following process:

- the strands were soaked for 1 hour in a solution of 10 wt.% Pro You™ Neutral Shampoo (commercially available from Revlon Professional) and tap water;
- the strands were rinsed for 30 seconds under tap water;
- excess water was removed with a towel; and
- finally, the strands were left to dry naturally.

2.2. Damaging (bleaching) hair

The strands of hair of step 2.1 were damaged according to the following process:

- the strands were soaked for 1 hour in a 26 wt.% hydrogen peroxide and 12.7 wt.% ammonia aqueous solution;
- the strands were rinsed for 5-10 minutes under tap water;
- the strands were soaked for 30 seconds in a solution of 10 wt.% Revlonissimo™ Post Color Shampoo (commercially available from Revlon Professional) and tap water, under agitation;
- the strands were rinsed for 30 seconds under tap water;
- excess water was removed with a towel; and
- finally, the strands were dried for 6 minutes using a Pro-Iron 3000 City dryer manufactured by Tectools and, after that, left to dry naturally.

2.3. Application

The compositions for treating keratin fibers C1-C3 and the comparative compositions CC1-CC6 of Ex. 1 were applied to the strands according to the following process:

2.3.1. Step 1

11 locks of hair from each strand (approximately 1.2 g each lock) were selected.
9 locks of hair were immersed for 10 minutes in each of the following solutions:

Comparative Composition CC1
Comparative Composition CC4
Composition C1
Comparative Composition CC2
Comparative Composition CC5
Composition C2
Comparative Composition CC3
Comparative Composition CC6
Composition C3

2 hair locks remained untreated.
All the hair locks were combed to untangle them and to spread the compositions uniformly.

2.3.2. Step 2

All hair locks were dried for 6 minutes using a Pro-Iron 3000 City dryer manufactured by Tectools. The hair locks were not combed.
All hair locks were straightened 5 times, 4 seconds each time, using a Titanium Ion Technology flat ironing manufactured by GA.MA S.r.l. The hair locks were not combed.

2.3.3. Step 3

Pictures were taken of each hair lock and the width of each hair lock was measured.

2.3.4. Step 4

Treated and untreated hair locks were kept at 25°C and 80% relative humidity in a HPP750 climatic chamber manufactured by Memmert GmbH & Co. KG.

2.3.4. Step 5

Pictures were taken of each hair lock and the width of each hair lock was measured at 24 h.
The frizz reduction factor (FRF) was calculated according to the following equation:

$$FRF = \frac{(\text{\% of width of the untreated hair lock - \% of width of the treated hair lock})}{(\text{\% of width of the untreated hair lock})} \times 100$$

Where

$$\% \text{ of width of the untreated hair lock} = \{[Wuh\ (24h) - Wuh\ (0h)] / Wuh\ (0h)\} \times 100$$

where,

Wuh (24h) represents the width of the untreated treated hair lock at 24h in the humidity chamber
Wuh (0h) represents the width of the untreated treated hair lock at 0h in the humidity chamber

$$\% \text{ of width of the treated hair lock} = \{[Wth\ (24h) - Wth\ (0h)] / Wth\ (0h)\} \times 100$$

where,

Wth (24h) represents the width of the treated hair lock at 24h in the humidity chamber
Wth (0h) represents the width of the treated hair lock at 0h in the humidity chamber

The results are shown in Table 3. Each value represents the mean of 5 hair locks.

Table 3 - Frizz Reduction Factor (FRF) of the compositions for treating keratin fibers C1-C3 and comparative compositions CC1-CC6 at 24 hours

| Compositions | FRF |
|---|---|
| CC1 | -17.05 |
| CC4 | -5.27 |
| C1 | 14.25 |
| CC2 | -61.94 |
| CC5 | -35.29 |
| C2 | 20.28 |
| CC3 | -36.14 |
| CC6 | -42.02 |
| C3 | 33.18 |

FRF positive values show a reduction in the frizz with respect to the untreated hair locks. FRF negative values show an increase in the frizz with respect to the untreated hair locks.

From the experimental results, it can be concluded that the compositions for treating keratin fibers of the present disclosure provide an anti-frizz effect to hair at least after 24 h of application. The compositions for treating keratin fibers also protect hair during heat treatments (heat-protection).

Modifications, which do not affect, alter, change or modify the essential aspects of the compositions and methods described above, are included within the scope of the present disclosure.

## Claims

1. A composition for treating keratin fibers comprising, in a cosmetically acceptable medium: (a) at least one film-forming amino-silicone polymer; and (b) at least one silane derivative of a hydrolyzed protein or peptide or amino acid.

2. The composition according to claim 1, wherein the at least one film-forming amino-silicone polymer is selected from Polysilicone-35, Polysilicone-33, Polysilicone-29, Polysilicone-28, Polysilicone-25, Polysilicone-24, Polysilicone-22, Polysilicone-19, Polysilicone-18, Polysilicone-14, Polysilicone-3, and mixtures thereof.

3. The composition according to claim 2, wherein the at least one film-forming amino-silicone polymer is selected from Polysilicone-35, Polysilicone-33, Polysilicone-29, and mixtures thereof.

4. The composition according to any one of claims 1 to 3, wherein the at least one silane derivative of a hydrolyzed protein or peptide or amino acid is selected from Hydrolyzed Collagen PG-Propyl Methylsilanediol, Hydrolyzed Keratin PG-Propyl Methylsilanediol, Hydrolyzed Sesame Protein PG-Propyl Methylsilanediol, Hydrolyzed Silk PG-Propyl Methylsilanediol, Hydrolyzed Soy Protein PG-Propyl Methylsilanediol, Hydrolyzed Wheat Protein PG-Propyl Methylsilanediol, Hydrolyzed Vegetable Protein PG-Propyl Silanetriol, Cystine Bis-PG-Propyl Silanetriol, and mixtures thereof.

5. The composition according to claim 4, wherein the at least one silane derivative of a hydrolyzed protein or peptide or amino acid is selected from Hydrolyzed Collagen PG-Propyl Methylsilanediol, Hydrolyzed Keratin PG-Propyl Methylsilanediol, Hydrolyzed Sesame Protein PG-Propyl Methylsilanediol, Hydrolyzed Silk PG-Propyl Methylsilanediol, Hydrolyzed Soy Protein PG-Propyl Methylsilanediol, Hydrolyzed Wheat Protein PG-Propyl Methylsilanediol, and mixtures thereof.

6. The composition according to any one of claims 1 to 5, wherein the total amount of the at least one film-forming amino-silicone polymer is in the range of about 0.05 to about 10 wt.%, based on the total weight of the composition.

7. The composition according to any one of claims 1 to 6, wherein the total amount of the at least one silane derivative of a hydrolyzed protein or peptide or amino acid is in the range of about 0.05 to about 10 wt.%, based on the total weight of the composition.

8. The composition according to claim 1 comprising, based on the total weight of the composition:

a) about 0.05-10 wt.% of the at least one film-forming amino-silicone polymer; and
b) about 0.05-10 wt.% of the at least one silane derivative of a hydrolyzed protein or peptide or amino acid.

9. The composition according to claim 8 comprising, based on the total weight of the composition:

a) about 0.08-5 wt.% of the at least one film-forming amino-silicone polymer; and
b) about 0.1-5 wt.% of the at least one silane derivative of a hydrolyzed protein or peptide or amino acid.

10. The composition according to any one of claims 8 or 9 comprising, based on the total weight of the composition:

a) about 0.05-10 wt.% of the at least one film-forming amino-silicone polymer selected from Polysilicone-35, Polysilicone-33, Polysilicone-29, Polysilicone-28, Polysilicone-25, Polysilicone-24, Polysilicone-22, Polysilicone-19, Polysilicone-18, Polysilicone-14, Polysilicone-3, and mixtures thereof; and
b) about 0.05-10 wt.% of the at least one silane derivative of a hydrolyzed protein or peptide or amino acid selected from Hydrolyzed Collagen PG-Propyl Methylsilanediol, Hydrolyzed Keratin PG-Propyl Methylsilanediol, Hydrolyzed Sesame Protein PG-Propyl Methylsilanediol, Hydrolyzed Silk PG-Propyl Methylsilanediol, Hydrolyzed Soy Protein PG-Propyl Methylsilanediol, Hydrolyzed Wheat Protein PG-Propyl Methylsilanediol, Hydrolyzed Vegetable Protein PG-Propyl Silanetriol, Cystine Bis-PG-Propyl Silanetriol, and mixtures thereof.

11. The composition according to claim 10 comprising, based on the total weight of the composition:

a) about 0.05-10 wt.% of the at least one film-forming amino-silicone polymer selected from Polysilicone-35, Polysilicone-33, Polysilicone-29, and mixtures thereof; and
b) about 0.05-10 wt.% of the at least one silane derivative of a hydrolyzed protein or peptide or amino acid selected from Hydrolyzed Collagen PG-Propyl Methylsilanediol, Hydrolyzed Keratin PG-Propyl Methylsilanediol, Hydrolyzed Sesame Protein PG-Propyl Methylsilanediol, Hydrolyzed Silk PG-Propyl Methylsilanediol, Hydrolyzed Soy Protein PG-Propyl Methylsilanediol, Hydrolyzed Wheat Protein PG-Propyl Methylsilanediol, and mixtures thereof.

12. The composition according to claim 10 comprising, based on the total weight of the composition:

a) about 0.08-5 wt.% of the at least one film-forming amino-silicone polymer selected from Polysilicone-35, Polysilicone-33, Polysilicone-29, and mixtures thereof; and
b) about 0.1-5 wt.% of the at least one silane derivative of a hydrolyzed protein or peptide or amino acid selected from Hydrolyzed Collagen PG-Propyl Methylsilanediol, Hydrolyzed Keratin PG-Propyl Methylsilanediol, Hydrolyzed Sesame Protein PG-Propyl Methylsilanediol, Hydrolyzed Silk PG-Propyl Methylsilanediol, Hydrolyzed

Soy Protein PG-Propyl Methylsilanediol, Hydrolyzed Wheat Protein PG-Propyl Methylsilanediol, and mixtures thereof.

**13.** A process for treating keratin fibers comprising the steps of:

i) applying to the keratin fibers a composition as defined in any one of claims 1 to 12;
ii) applying heat to the keratin fibers treated in step (i).

**14.** Use of a composition as defined in any one of claims 1 to 12 to protect keratin fibers from heat damage.

**15.** Use of a composition as defined in any one of claims 1 to 12 as an anti-frizz agent for keratin fibers.

Europäisches Patentamt
European Patent Office
Office européen des brevets

# EUROPEAN SEARCH REPORT

Application Number
EP 20 38 2555

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE GNPD [Online] MINTEL; 26 March 2015 (2015-03-26), anonymous: "Damage Repair Shampoo", XP055642900, retrieved from www.gnpd.com Database accession no. 3068005 * Ingredients; page 2 * | 1,13 | INV. A61K8/58 A61K8/898 A61Q5/00 A61Q5/04 |
| X | WO 2019/207447 A1 (SIVLER PETTER [SE]; RAVICHANDRAN RANJITHKUMAR [SE]) 31 October 2019 (2019-10-31) * example 8 * * claim 10 * | 1,13 | |
| Y | CN 110 934 781 A (ZHAOQING DICAI DAILY CHEMICAL SCIENCE AND TECH CO LTD) 31 March 2020 (2020-03-31) | 1,13 | |
| A | * page 3, paragraphs 1,10; claim 1 * * example 1 * | 2-12,14, 15 | |
| Y | EP 3 659 577 A1 (KAO GERMANY GMBH [DE]) 3 June 2020 (2020-06-03) | 1,13 | TECHNICAL FIELDS SEARCHED (IPC) A61K A61Q |
| A | * claim 1; examples 1,3 * | 2-12,14, 15 | |

The present search report has been drawn up for all claims

2

| Place of search | Date of completion of the search | Examiner |
|---|---|---|
| Munich | 30 November 2020 | Tullberg, Erik |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 20 38 2555

This annex lists the patent family members relating to the patent documents cited in the above-mentioned European search report. The members are as contained in the European Patent Office EDP file on The European Patent Office is in no way liable for these particulars which are merely given for the purpose of information.

30-11-2020

| Patent document cited in search report | | Publication date | Patent family member(s) | | Publication date |
|---|---|---|---|---|---|
| WO 2019207447 | A1 | 31-10-2019 | AU | 2019259637 A1 | 26-11-2020 |
| | | | CA | 3096437 A1 | 31-10-2019 |
| | | | WO | 2019207447 A1 | 31-10-2019 |
| CN 110934781 | A | 31-03-2020 | NONE | | |
| EP 3659577 | A1 | 03-06-2020 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- CTFA dictionary. 2004
- Hair Preparations. **THOMAS CLAUSEN et al.** Ullmann's Encyclopedia of Industrial Chemistry. 2006